# FASCICULE DE BREVET EUROPEEN

(11) **EP 0 858 325 B1**
(45) Date de publication et mention de la délivrance du brevet: **31.07.2002**
(21) Numéro de dépôt: 96937367.9
(22) Date de dépôt: 30.10.1996
(51) Int. Cl.: A61K 9/19, A61K 9/14, A61K 47/26, A61K 47/18

(54) **FORMULATION PHARMACEUTIQUE LYOPHILISEE STABLE**
LYOPHILISIERTE STABILE PHARMAZEUTISCHE FORMULIERUNG
STABLE FREEZE-DRIED PHARMACEUTICAL FORMULATION

(30) Priorité: 03.11.1995 FR 9513022
(43) Date de publication de la demande: 19.08.1998
(73) Titulaire: SANOFI-SYNTHELABO, 75635 Paris Cédex 13 (FR)
(72) Inventeur: BOULOUMIE, Colette, Bâtiment 5, F-34000 Montpellier (FR); BREUL, Thierry, F-34080 Montpellier (FR); COLLIERE, Laurence, F-82700 Montbartier (FR); FAURE, Philippe, F-34970 Maurin (FR)
(74) Mandataire: Bernasconi, Jean Raymond
(86) Numéro de dépôt international: FR9601706
(87) Numéro de publication internationale: WO9717064

(56) Documents cités:
- EP-A- 0 394 045
- EP-A- 0 682 944
- WO-A-93/23017
- GB-A- 2 021 581
- US-A- 4 537 883
- CHEMICAL ABSTRACTS, vol. 83, no. 22, 1 Décembre 1975 Columbus, Ohio, US; abstract no. 183393, TSUNAKAWA N. ET AL: "Freeze drying of panthetine" XP002009310 & DATABASE WPI Week 7552 Derwent Publications Ltd., London, GB; AN 75-85480w & JP 50 088 215 A (DAICHI PHARM. KK) , 15 Juillet 1975
- DATABASE WPI Section Ch, Week 9020 Derwent Publications Ltd., London, GB; Class B05, AN 90-151822 XP002009311 & JP 02 096 536 A (GREEN CROSS CORP.) , 9 Avril 1990 & PATENT ABSTRACTS OF JAPAN vol. 14, no. 294 (C-732), 26 Juin 1990 & JP 02 096536 A (GREEN CROSS CORP.), 9 Avril 1990,

## Description

La présente invention concerne une formulation pharmaceutique se présentant sous forme de lyophilisat et contenant au moins un pnncipe actif de nature non protéique. Plus particulièrement. l'invention concerne une telle formulation, stable à des températures qui peuvent aller de 25°C à 40°C, qui peut être soit reconstituée sous forme liquide par ajout d'un solvant pour son administration par voie parentérale ou orale, soit directement administrée par voie orale, à l'homme ou à l'animal.

Le principe actif contenu dans la formulation selon l'invention pourra être seul ou bien associé a un autre principe actif de nature protéique ou non protéique.

On sait que la lyophilisation peut avoir un effet considérable sur la dégradation des principes actifs pharmaceutiques dans une formulation, ainsi qu'un fort impact sur leur stabilité sous forme lyophilisée. Les diverses vanables qui affectent ces paramètres sont principalement le pH, la quantité de sels présents, le type et la quantité d'excipients dans la formulation, le type de cryoprotection choisi, ainsi que les températures, pression et temps des opérations de congélation, sublimation et dessiccation choisis. Ces différentes variables influent sur l'état physique du lyophilisat obtenu, à savoir : amorphe vitreux, amorphe mou, cristallin ou une combinaison de ces états.

Pour la conservation des lyophilisats, on recourt souvent à des acides aminés, de préférence à la glycine, et à des polyols, de préférence au mannitol, mais la littérature, très copieuse sur le sujet, ne donne aucun renseignement sur la solution du problème général de l'obtention d'une formulation pharmaceutique stable qui tienne compte des différents paramètres qui interviennent dans les opérations de formulation et de lyophilisation d'un principe actif non protéique en association avec un acide aminé et un polyol.

Plus particulièrement, la littérature enseigne que la présence d'un acide aminé, d'un polyol, par exemple le mannitol, d'une phase cristalline ou d'une phase amorphe peut comporter, à côté d'avantages certains, des inconvénients qui se traduisent, dans le cas de lyophilisats contenant des principes actifs particulièrement sensibles. par des délais de péremption relativement courts et/ou des températures de stockage de ces lyophilisats inférieures à 8°C. Il serait pourtant particulièrement avantageux, notamment pour un traitement ambulatoire, de pouvoir obtenir une formulation stable à température ambiante jusqu'à sa reconstitution et ainsi d'éviter sa conservation au réfrigérateur avant et en cours de traitement.

Le rôle du polyol et de l'acide aminé a été étudié séparément dans le cas de l'hormone de croissance humaine (hGH), mais leur effet synergique est encore mal élucidé (Pikal M.J., Dellermann K.M., Roy M.L. Riggin M.N., The effects of formulation variables on the stability of freeze-dried Human Growth Hormone, Pharm. research., 1991, *8*, N° 4, 427-436).

Les avantages et les inconvénients liés à la présence d'acides aminés, de mannitol, d'une phase cristalline ou d'une phase amorphe sont répertoriés ci-après.

Avantages liés à la présence d'acides aminés.

Il a été démontré que la présence de glycine dans un lyophilisat, induisait une cristallisation des molécules présentes en solution au cours de l'étape de congélation de la lyophilisation (Korey D.J., Schwartz J.B., Effects of excipients on the cristallization of pharmaceutical compounds during lyophylization, J. Parenteral Sci. Tech., 1989, *43*, *2*, 80-83). Cette cristallisation du principe actif permet d'améliorer sa stabilité.

L'alanine sous forme cristallisée a l'avantage d'empêcher l'affaissement du lyophilisat en cours de sublimation et de dessiccation et de permettre l'obtention d'un lyophilisât d'une surface spécifique plus importante et permet donc une dessiccation plus rapide (Pikal M.J., Freeze-drying of proteins, Biopharm., 26-30 october 1990).

Inconvénients liés à la présence d'acides aminés.

L'ajout d'un acide aminé à un sucre ou à un polyol dans une solution à lyophiliser a généralement pour effet de diminuer la température de transition vitreuse du sucre (te Booy M.P.W.M., de Ruiter R.A., de Meere A.L.J., Evaluation of the physical stability of freeze-dried sucrose containing formulations by differential scanning calorimetry, Pharm. Research., 1992, *9*, 109-114). Or un abaissement de la température de transition vitreuse est généralement synonyme de moindre stabilité d'un lyophilisat (Franks F., Freeze-drying ; from empiricism to predictability, Cryo-letters, 1990, *11*, 93-110).

Avantages liés à la présence de mannitol.

La présence de mannitol dans la composition d'un lyophilisat est généralement justifiée en tant que ballast de lyophilisation, c'est à dire qu'il permet à la fois de maintenir une structure solide et rigide du volume du lyophilisât correspondant au volume de solution à lyophiliser, mais sa présence permet aussi d'ajuster à l'isotonie la solution reconstituée à injecter. Lorsque le mannitol est l'excipient majoritaire dans la composition d'un lyophilisat, il est le plus souvent sous forme cristalline (Lyophilized formulations recombinant tumor necrosis factor, Hora M.S., Rana R.K., Smith F.W., Pharm. Res., 1992, *9* (1), 33-36).

Inconvénients liés à la présence du mannitol.

II a été reporté que le taux d'hydrolyse du méthylprednisolone sodium succinate sous forme lyophilisée était plus important en présence de mannitol qu'en présence de lactose, et que ce taux augmentait avec la quantité de mannitol présente dans le lyophilisat. Ceci a été expliqué par le fait que la cristallisation du mannitol en cours de lyophilisation change la distribution de l'eau dans la matrice du lyophilisat. L'accroissement de la quantité d'eau présente dans le micro-environnement du principe actif qui en résulte, favorise l'hydrolyse du principe actif et diminue sa stabilité (The effect of bulking agent on the solid state stability of freeze dried methylprednisolone sodium succinate, Herman B.D., Sinclair B.D., Milton N., Nail S.L., Pharma. Res., 1994, *11* (10), 1467-1473).

Avantages liés à la présence d'une phase cristalline.

La présence d'un soluté cristallisé, dans une solution congelée est un moyen de stabiliser les protéines en cours de dessiccation (Carpenter J.F. & Crowe J.H., Modes of stabilization of a protein by organic solutes during dessiccation, Cryobiology, 1988, *25*, 459-470). De plus la cristallisation, en cours de congélation, des excipients majoritairement présents dans une solution à lyophiliser, rend plus efficace les opérations de sublimation et de dessiccation secondaires, en augmentant la surface spécifique d'échange entre l'atmosphère de l'enceinte du lyophilisateur et le solide à sublimer. Cette augmentation de surface spécifique des formes cristallines par rapport aux formes amorphes facilite les échanges thermiques en cours de lyophilisation. La conséquence de cette efficacité accrue de la lyophilisation est l'obtention de formes lyophilisées dont la teneur en eau résiduelle est moins élevée, ce qui implique une stabilité accrue du lyophilisat à des températures plus élevées (Korey D.J., Schwartz J.B., Effects of excipients on the cristallization of pharmaceutical compounds during lyophylization, J. Parenteral Sci. Tech., 1989, *43*, *2*, 80-83).

Inconvénients liés à la présence d'une phase cristalline.

En général les substances cristallisées ont des vitesses de dissolution moins rapide que les substances amorphes. En effet, il faut plus d'énergie pour arracher une molécule à un réseau organisé d'un arrangement cristallin, que pour l'arracher à l'assemblage inorganisé d'un état amorphe. Parfois la vitesse de dissolution devient insuffisante pour permettre une absorption suffisamment rapide de ces substances, pouvant entrainer une diminution de leur activité, spécialement dans le cadre de molécules peu stables en solution. De la même manière, la parfaite régularité des cristaux étant un cas idéal, l'hétérogénéité de la phase cristalline et le polymorphisme obtenus pour une même substance et entre substances associées induisent des vitesses de dissolutions différentes pour une même substance et entre chacune des substances, pouvant aboutir à des effets thérapeutiques non reproductibles (Galénica 2, Biopharmacie 2ème édition, 1982, technique et documentation).

En outre, il a été démontré que la perte d'activité d'une protéine lyophilisée était directement reliée aux taux de cristallinité de la molécule cryoprotectrice (lzutsu K.L., Yoshioka S., Terao T., Decreased protein-stabilizing effects of cryoprotectants due to crystallization., Pharm. Research. 1993, *10*, N° 8, 1232-1237 ; Izutsu K.I., Yoshioka S., Kojima S., Increased stabilizing effects of amphiphilic excipients on freeze drying of lactate deshydrogenase (LDH) by dispersion into sugar matrixes, Pharm. Res., 1995, *12* (6), 838-843). Dans la formulation des médicaments contenant des protéines, la cristallisation des excipients doit être évitée selon : (Hermansky M., Pesak M., Lyophilization of drugs. VI Amorphous and Cristalline forms Cesk. Farm., 1993, *42*, (2), 95-98).

Avantages liés à la présence d'une phase amorphe.

Dans le même ordre d'idée, la forme amorphe se dissout plus rapidement que la forme cristallisée et ne présente pas les inconvénients liés à l'hétérogénéité et au polymorphisme des substances cristallisées.

D'autre part, la présence d'additifs à l'état amorphe stabilise l'activité de certaines enzymes proportionnellement à la concentration de l'additif selon lzutsu K.L., Yoshioka S., Terao T., Decreased protein-stabilizing effects of cryoprotectants due to crystallization., Pharm. Research., 1993, *10*, N° 8, 1232-1237.

L'effet cryoprotecteur des excipients est attribué à l'état amorphe de la glycine dans le lyophilisat obtenu (Pikal M.J., Dellermann K.M., Roy M.L. Riggin M.N., The effects of formulation variables on the stability of freeze-dried Human Growth Hormone, Pharm. Research., 1991, *8*, N° 4, 427-436).

Inconvénients liés à la présence d'une phase amorphe.

En présence d'une phase amorphe solide seule, le lyophilisat s'affaisse aux températures supérieures à la température de transition vitreuse en cours de congélation.

Au sein d'une phase amorphe molle les réactions chimiques de dégradation ont une cinétique beaucoup plus rapide qu'au sein d'une phase cristalline (Solid state stability and preformulation study of a new parenteral cephalosporin antibiotics (E1040), Ashizawa K., Uchikawa K., Hattori T., Ishibashi Y., Miyake Y., Sato T., Yakugaku Zasshi, 1990, *110*(3), 191-201).

De plus, la plus grande vitesse de dissolution des substances amorphes s'accompagne parfois d'une plus grande instabilité, la transformation d'une forme se faisant en général de l'état amorphe à l'état cristallisé (Galénica 2, Biopharmacie 2ème édition, 1982, technique et documentation).

En conclusion, la littérature scientifique, au sujet de l'effet des excipients sur la stabilisation des principes actifs pharmaceutiques, donne des informations contradictoires sur leurs propriétés, et ne permet pas non plus d'obtenir des informations certaines au sujet des relations entre la structure d'un lyophilisat et sa stabilité. De même, le rôle des polyols et des acides aminés, seuls ou en association, n'est pas décrit selon un ensemble de propriétés généralisables, mais a été observé avec des résultats contradictoires selon les principes actifs étudiés et les quantités d'excipients mises en jeu.

WO 93/23017 a trait à des unités solides de dosage facilement dispersables dans l'eau, comprenant une matrice poreuse contenant, à titre d'ingrédients essentiels :
- un agent formant matrice choisi parmi les gélatines, pectines, protéines extraites de fibres de soja et leurs mélanges ;
- un ou plusieurs aminoacides ; et
- éventuellement un glucide.

Les formulations lyophilisées de l'invention diffèrent des compositions de WO 93/12017 en ce qu'elles ne contiennent pas l'agent formant matrice, indispensable selon l'art antérieur.

Il a maintenant été trouvé qu'il existe un effet synergique entre le mannitol et l'alanine sur la stabilisation des principes actifs pharmaceutiques lyophilisés. It a été notamment démontré que cet effet synergique existe seulement dans un domaine étroit de concentrations relatives de chacun de ces deux excipients.

A la base de la présente invention il y a notamment la découverte d'un effet synergique surprenant résultant de la coexistence d'une phase amorphe et d'une phase cristalline qui a pour conséquence de stabiliser le principe actif pharmaceutique lyophilisé. La présente invention décrit donc l'obtention de cet effet pour des rapports mannitol / alanine privilégiés.

Ainsi la présente invention concerne une formulation pharmaceutique lyophilisée constituée d'une phase amorphe et d'une phase cristalline, comprenant une quantité efficace d'au moins un principe actif pharmaceutique non protéique, du mannitol et de l'alanine, ces deux derniers excipients étant dans un rapport massique R compris entre 0,1 et 1, R étant le rapport entre la masse du mannitol et la masse de l'alanine.

Le principe actif inclus dans ladite formulation reste stable à des températures qui peuvent aller de 25°C à 40°C sous forme lyophilisée. Le cas échéant la dissolution du lyophilisat obtenu est rapide et totale. L'aspect du lyophilisat n'est pas effondré et sa teneur en eau est compatible avec le maintien de la stabilité du principe actif.

Il a été démontré que, pour R compris entre 0,1 et 1:
- le lyophilisat est constitué d'une phase amorphe et d'une phase cristalline,
- la phase amorphe est majoritairement constituée de mannitol et de principe actif,
- la phase cristalline est majoritairement constituée d'alanine.

Bien que l'invention ne soit pas limitée à une théorie particulière rendant compte de la stabilisation obtenue par association d'un ou plusieurs principes actifs non protéiques, de mannitol et d'alanine dans les rapports indiqués, on peut émettre l'hypothèse suivante:
- la phase amorphe, mise en évidence par l'analyse thermique différentielle, cryoprotège le principe actif pharmaceutique en cours de congélation, le principe actif étant lui-même dispersé dans cette forme amorphe, et la phase cristalline, mise en évidence par diffractométrie des rayons X, fixe la structure du lyophilisat et évite son effondrement.

Selon un autre de ces aspects, la présente invention a pour objet l'obtention des lyophilisats stables contenant un principe actif pharmaceutique cryoprotégé par une phase solide amorphe constituée complètement ou partiellement par du mannitol, cette phase amorphe coexistant au sein du lyophilisat obtenu après sublimation et dessiccation de la solution congelée, avec une phase cristalline constituée essentiellement par l'alanine.

Ainsi, la présente invention a également pour objet un procédé pour la préparation de formulations pharmaceutiques lyophilisées comprenant au moins un principe actif non protéique caractérisé en ce qu'on lyophilise un mélange dudit principe actif, du mannitol et d'alanine dans lequel le mannitol et l'alanine sont présents dans le rapport R compris entre 0,1 et 1, R étant le rapport entre les masses du mannitol et de l'alanine.

D'autres excipients pharmaceutiquement acceptables, normalement utilisés dans les formes lyophilisées, peuvent être introduits dans la formulation selon la présente invention, comme par exemple des tampons ou des acides-bases permettant d'ajuster le pH, des tensio-actifs, des sels, des conservateurs, notamment des conservateurs antibactériens, des anti-oxydants ou des agents chélatants à l'exclusion des excipients qui, dans le lyophilisat contenant le principe actif, empêcheraient la coexistence de la phase amorphe constituée majoritairement par le mannitol et de la phase cristalline constituée majoritairement par l'alanine, comme par exemple certains dérivés protéiques d'origine animale ou végétale tels que les gélatines, les dextrines ou les protéines extraites de graines de blé ou de soja, les gommes telles que l'agar ou le xanthane, les polyssacharides, les alginates, les carboxyméthylcelluloses, les pectines, les polymères synthétiques comme la polyvinylpyrrolidone ou des complexes de nature polyssacharidiques comme la gélatine d'acacia. Parmi les tampons qui peuvent être introduits dans la formulation selon la présente invention, on citera en particulier les tampons carbonate, borate, phosphate, citrate, tri(hydroxyméthyl)aminométhane, maléate et tartrate, les acides ou les bases constituant ces tampons pouvant également être introduits seuls.

Parmi les tensio-actifs qui peuvent être introduits dans la formulation selon la présente invention, on citera en particulier les polysorbates, les poloxamers, le tyloxapol, les lécithines. Parmi les sels qui peuvent être introduits dans la formulation selon la présente invention, on citera en particulier les sels de sodium comme l'édédate (EDTA tétrasodique), le chlorure, le docusate (1,4-bis(2-éthylhexyl)sulfosuccinate de sodium), le bicarbonate, le glutamate ; l'acétate de potassium ; le carbonate dipotassique et le stéarate de magnésium.

Parmi les conservateurs qui peuvent être introduits dans la formulation selon la présente invention, on citera en particulier les parahydroxybenzoate de méthyle et propyle, le chlorure de benzéthonium, le mercurothiolate de sodium, le nitratephénylmercure, l'alcool benzylique, le phénol et le métacrésol.

La coexistence de la phase mannitol amorphe et de la phase alanine cristalline est indépendante de la présence et de la concentration du tampon utilisé pour ajuster le pH de la solution, mais elle dépend du rapport R précédemment défini.

Des exemples de formulation des solutions à lyophiliser conduisant aux formulations de l'invention sont les suivantes :

Un ou une association de principes actifs pharmaceutiques, un tampon pharmaceutiquement acceptable pour ajuster le pH, du mannitol et de l'alanine avec un rapport massique R = masse de mannitol/masse d'alanine compris entre 0,1 et 1, de l'eau pour préparations injectables, ainsi que, si nécessaire, des conservateurs antibactériens et les excipients permettant la solubilisation du ou des principes actifs.

Selon un mode de réalisation préféré de l'invention, le mélange alanine/mannitol est majoritaire.

La quantité de principe actif présent est limitée par sa solubilité dans l'eau. Les formulations de l'invention résultent en effet de la lyophilisation de solutions aqueuses dans lesquelles le principe actif est parfaitement dissous.

De même tout excipient est présent dans la formulation en quantité inférieure à la quantité du mélange alanine/mannitol.

Les solutions à lyophiliser se préparent de la façon suivante :

Les quantités désirées de tampon, d'alanine, de mannitol, de conservateurs et de principe actif sont ajoutées à la température de dissolution appropriée à la quantité d'eau pour préparations injectables ou d'agent solubilisant nécessaire à leur solubilisation jusqu'à complète dissolution. Les solutions obtenues sont filtrées en milieu stérile et réparties en récipients, préférentiellement des flacons ou des carpules.

La lyophilisation des solutions est réalisée comme suit :

La solution suit un cycle de congélation, puis de sublimation et de dessiccation adapté au volume à lyophiliser et au récipient contenant la solution.

Préférentiellement on choisit une vitesse de congélation proche de -2°C/mn dans un lyophilisateur Usifroid (France) de type SMH15, SMJ100 ou SMH2000.

Les temps, les températures et les pressions de sublimation et de dessiccation sont ajustés en fonction des volumes de solution à lyophiliser et de la teneur en eau résiduelle désirée dans le lyophilisât.

On obtient alors un lyophilisat dans lequel l'alanine se trouve sous forme cristallisée, et le mannitol sous forme complètement ou partiellement amorphe. Le lyophilisat peut être conservé à 25°C et même jusqu'à 40°C sans altérer la stabilité chimique et biologique du principe actif qu'il contient.

Une information complète sur les techniques de préparation des formulations injectables par dissolution des compositions de l'invention est à la disposition de l'homme du métier dans Remington's Pharmaceutical Sciences, 1985, 17th Edition ou dans William N.A. & Polli G.P., The lyophilization of pharmaceuticals : a litterature review, J. Parenteral Sci. Tech., 1984, *38*, (2), 48-59 ou dans Franks F., Freeze-drying : from empiricism to predictability, Cryo-letters, 1990, *11*, 93-110.

Le principe actif ou les principes actifs associés, de type non protéique, formulés selon la présente invention pourront être des antalgiques, des anti-inflammatoires, des antispasmodiques, des anti-cancéreux ou des principes actifs utilisables en cardiologie, angiologie, gastro-entérologie, hématologie et hémostase, hépatologie, infectiologie, neurologie-psychiatrie, rhinologie, rhumatologie, toxicologie, urologie, ou dans le domaine du diagnostic ou en tant que régulateurs du métabolisme et de la nutrition.

Dans les familles thérapeutiques et domaine d'activité biologique mentionnés ci-dessus à titre exemplificatif, n'importe quel produit peut constituer le principe actif des formulations de la présente invention qui représentent un progrès technique considérable dans la technique pharmaceutique. De préférence, les principes actifs les plus adaptés aux formulations de la présente invention sont ceux dont la stabilité en solution aqueuse est problématique. Il est cependant envisageable d'appliquer la présente invention à des principes actifs qui n'ont pas de problème particulier de stabilité.

Dans la suite, on a adopté les dénominations communes internationales pour désigner les principes actifs.

Le principe actif des formulations pharmaceutiques lyophilisées de la présente invention peut être choisi notamment parmi le groupe constitué par :
- les acides phénylalcanoïques, par exemple le kétoprofène ;
- les anti-inflammatoires non-stéroïdiens du type "oxicam", par exemple piroxicam, isoxicam, ténoxicam ;
- le paracétamol ;
- l'acétylsalicylate de lysine ou d'arginine ;
- les corticostéroïdes, par exemple la méthylprednisolone ;
- le phloroglucinol ;
- les acides biliaires, par exemple l'acide ursodésoxycholique ou un de ses sels pharmaceutiquement acceptables avec des bases inorganiques ou organiques, de préférence son sel de sodium ;
- les anthracyclines, par exemple la doxorubicine, l'épirubicine, l'idarubicine, la daunorubicine, la pirarubicine ;
- les dérivés de platine, par exemple la cisplatine, l'oxaliplatine, la carboplatine ;
- les dérivés des alcaloïdes de la vinca minor, par exemple la vinblastine, la vincristine ;
- les dérivés des alcaloïdes de l'ergot de seigle, par exemple la dihydroergotamine, la dihydroergotoxine, la nicergoline;
- les dérivés des bases puriques ou pyrimidiques, par exemple l'acyclovir, le gancyclovir, la cytarabine;
- les prostaglandines, par exemple la sulprostone, l'alprostadil ;
- les benzodiazépines, par exemple le clorazépate dipotassique, le dévazépide
- les antibiotiques bêta-lactamiques, par exemple la pipéracilline, le tazobactam;
- les antibiotiques macrolides, par exemple l'érythromycine ou un de ses dérivés, en général une leucomycine;
- les antibiotiques de la famille des tétracyclines, par exemple la minocycline;
- les antibiotiques du type chloramphénicol, par exemple le thiamphénicol;
- tes antibiotiques du type spiramycine;
- les moutardes azotées, par exemple le chlorambucil et les nitroso-urées, par exemple la carmustine et la streptozocine. Les moutardes azotées et les nitroso-urées sont décrites plus en détail dans Pharmacologie de M. Schorderet et coll. 1992, 2e édition, chapitre 69, Ed. Frison. Roche, Paris;
- les H₂-antagonistes, par exemple la ranitidine, la famotidine ou un de leurs sels pharmaceutiquement acceptables ;
- l'oméprazole et ses analogues ;
- les vitamines, par exemple la thiamine, la riboflavine, la nicotinamide, la pyridoxine, le panthoténate de sodium, la biotine, l'acide ascorbique, l'acide folique, la cyanocobalamine, le rétinol, le cholécalciférol, l'alphatocophérol, la cobalamide, l'hydroxycobalamide ;
- les antitumoraux choisis parmi le taxol, le taxotère et leurs analogues, la dacarbazine, le méthotréxate, la plicamycine, le thiotépa, la streptozocine;
- les médicaments cardiovasculaires choisis parmi la molsidomine ou un de ses sels pharmaceutiquement acceptables, notamment son chlorhydrate, la linsidomine, l'acétazolamide, le méclofénoxate, le diltiazem, le nitroprussiate de sodium ;
- les médicaments hématologiques choisis parmi la ticlopidine ou un de ses sels pharmaceutiquement acceptables, notamment son chlorhydrate, le molgramostim, l'acide folinique;
- les médicaments anticoagulants et antithrombotiques choisis parmi l'héparine, l'héparine de bas poids moléculaire sous forme de nadroparine calcique, pamaparine sodique, daltéparine sodique, énoxaparine sodique, ardéparine sodique, certoparine sodique, réviparine sodique, minoltéparine sodique, les pentasaccharides antithrombotiques naturels ou de synthèse ;
- les héparinoïdes, par exemple le lomoparan ;
- l'oxoglutarate de di-arginine et les sels pharmaceutiquement acceptables de l'acide oxoglutarique ;
- les extraits de plantes, par exemple à base de saule, d'harpagophytum, de ginseng, de fucus ;
- un gène, un fragment d'ADN ou d'ARN destiné à la thérapie génique, un oligonucléotide, un oligonucléotide antisense, des nucléotides associés à des composés protéiques comme par exemple des extraits de fractions de ribosomes, des virus vivants atténués ou inactivés ;
- l'acide valproïque et ses analogues ;
- la métopimazine ;
- la moxisylite;
- le pralidoxime ;
- la déféroxamine;
- le phénobarbital ou autres barbituriques ;
- le clométhiazole ;
- le pamidronate de sodium, l'alandronate de sodium, le risendronate de sodium et autres biphosphonates actifs en tant qu'agent antiostéoporotique, notamment le tiludronate ou sel disodique du {[(4-chlorophényl)thio]méthylène}bis (phosphonate) (SR 41319) sous forme hémihydratée ou monohydratée;
- les antagonistes 5-HT₂, notamment la kétansérine, la ritansérine, le (1Z,2E)-1-(2-fluorophényl)-3-(4-hydroxyphényl)-prop-2-èn-1-one-O-(2-diméthylaminoéthyl)oxime (SR 46349) ou un de ses sels pharmaceutiquement acceptables ;
- les antagonistes de l'angiotensine II, notamment le tasosartan, le telmisartan, le losartan potassium, le losartan associé à l'hydrochlorothiazide (HCTZ), l'éprosartan, le candésartan cilexétil, le valsartan, l'irbésartan ou 2-n-butyl-3-{[2'-(1*H*-tétrazol-5-yl)biphényl-4-yl]méthyl}-1,3-diazaspiro[4,4]non-1-èn-4-one (SR 47436) et ses sels pharmaceutiquement acceptables ;
- la fantofarone ou le 1-[(p-{3-[(3,4-diméthoxyphénéthyl)méthylamino] propoxy}phényl)sulfonyl]-2-isopropylindolizine et ses sels pharmaceutiquement acceptables ;
- la tirapazamine ou le 3-amino-1,2,4-benzotriazine-1,4-dioxyde et ses sels pharmaceutiquement acceptables ;
- le (2S)-1-[(2R,3S) 5-chloro-3-(2-chlorophényl)-1-(3,4-diméthoxybenzènesulfonyl)-3-hydroxy-2,3-dihydro-1*H*-indole-2-carbonyl]pyrrolidine-2-carboxamide (SR 49059) et ses sels pharmaceutiquement acceptables ;
- le N,N-dibutyl-3-{4-[(2-butyl-5-méthylsulfonamido)benzofuran-3-yl-carponyl] phénoxyl}propylamine et ses sels pharmaceutiquement acceptables, notamment le chlorhydrate (SR 33589);
- le 6-(2-diéthylamino-2-méthyl)propylamino-3-phényl-4-propylpyridazine (SR 46559) et ses sels pharmaceutiquement acceptables;
- l'éthyl{(7S)-7-[(2R)-2-(3-chlorophényl)-2-hydroxyéthylamino]-5,6,7,8-tétrahydronaphthalèn-2-yloxy}acétate et ses sels pharmaceutiquement acceptables, notamment le chlorhydrate (SR 58611A);
- le 1-(2,4-dichlorophényl)-3-(N-pipéridin-1-yl-carboxamido)-4-méthyl-5-(4-chlorophényl)-*1H*-pyrazole et ses sels pharmaceutiquement acceptables, notamment le chlorhydrate (SR 141716A);
- le 4-{[N-(3,4-diméthoxy-phénéthyl)]-N-méthylaminopropoxyl}-2-benzènesulfonyl-3-isopropyl-1-méthyl-indole (SR 33805) et ses sels pharmaceutiquement acceptables ;
- l'acide 2-{[1-(7-chloroquinolin-4-yl)-5-(2,6-diméthoxyphényl)-1*H*-pyrazole-3-carbonyl] amino}adamantane-2-carboxylique (SR 48692) et ses sels pharmaceutiquement acceptables ;
- le N-cyclohexyl-N-éthyl-3-(3-chloro-4-cyclohexylphényl)prop-2-énylamine (SR 31747);
- le (-)-N-methyl-N-[4-(4-acétylamino-4-phénylpipéridino)-2-(3,4-dichlorophényl)butyl] benzamide (SR 48968) et ses sels pharmaceutiquement acceptables ;
- le chlorure de (S)-1-{2-[3-(3,4-dichlorophényl)-1-(3-isopropoxyphénylacétyl) pipéridin-3-yl]éthyl}-4-phényl-1-azoniabicyclo[2.2.2] octane (SR 140333A) et ses sels quaternaires pharmaceutiquement acceptables, par exemple, le benzènesulfonate ;
- le 4-amino-1-(6-chloropyrid-2-yl)pipéridine et ses sels pharmaceutiquement acceptables, notamment le chlorhydrate (SR 57227A);
- le (S)-N-(1-{3-[1-benzoyl-3-(3,4-dichlorophényl)pipéridin-3-yl]propyl}-4-phényl pipéridin-4-yl)-N-méthylacétamide (SR 142801) et ses sels pharmaceutiquement acceptables ;
- l'acide 2-{[4-(2-chlorophényl)thiazol-2-yl]aminocarbonyl}indole-1-acétique (SR 27897)
   et ses sels pharmaceutiquement acceptables ;
- le clopidogrel ou le (+)-(S)-α-(2-chlorophényl)-4,5,6,7-tétrahydrothiéno-[3,2-c]pyridine -5(4*H*)-acétate de méthyle et ses sels pharmaceutiquement acceptables, notamment son hydrogènosulfate ;
- le chlorhydrate de 1-(2-naphtalèn-2-yléthyl)-4-(3-trifluorométhylphényl)-1,2,3,6-tétrahydropyridine (SR 57746A) et ses sels pharmaceutiquement acceptables, notamment son chlorhydrate ;
- la N,N-diméthyl-N'-(pyridin-3-yl)méthyl-N'-[4-(2,4,6-triisopropylphényl)thiazol-2-yl] éthane-1,2-diamine et ses sels pharmaceutiquement acceptables, notamment le fumarate (SR 27417) ;
- l'acide 2-[(5-(2,6-diméthoxyphényl)-1-{4-[(3-diméthylaminopropyl)méthylcarbamoyl]-2-isopropyl-phény}-1*H*-pyrazole-3-carbonyl)amino]adamantane-2-carboxylique et ses sels pharmaceutiquement acceptables (SR 142948A);
- le 3-(1-{2-[4-benzoyl-2-(3,4-difluorophényl)morpholino-2-yl]éthyl}-4-phénylpipéridin-4-yl)-1,1-diméthylurée et ses sels pharmaceutiquement acceptables (SR 144190A);
- le trichlorhydrate de l'acide 3-[N-{4-[4-(aminoiminométhyl)phényl]-1,3-thiazol-2-yl}
- N-(1-carboxyméthylpipéridin-4-yl)amino]propionique et ses sels pharmaceutiquement acceptables (SR 121566) ;
- le 3-[N-{4-[4-(amino(N-éthoxycarbonyl-imino)méthyl)phényl]-1,3-thiazol-2-yl}-N-(1-éthoxycarbonylméthyl)pipéridin-4-yl)amino]propionate d'éthyle (SR 121787) et ses sels pharmaceutiquement acceptables ;
- le 5-éthoxy-1-[4-(N-*ter*-butylcarbamoyl)-2-méthoxybenzènesulfonyl]-3-spiro-[4-(2-morpholinoéthyloxy)cyclohexane]indolin-2-one (SR 121463) et ses sels pharmaceutiquement acceptables.

On préfère tout particulièrement les formulations de l'invention dans lesquelles le principe actif est choisi parmi l'acide 2-{[4-(2-chlorophényl)thiazol-2-yl]aminocarbonyl}indole-1-acétique ou son sel de potassium, l'irbésartan, le clopidogrel, l'acide ursodésoxycholique et son sel de sodium, le chlorhydrate de 1-(2-naphtalèn-2-yléthyl)-4-(3-trifluorométhylphényl)-1,2,3,6-tétrahydropyridine, le fumarate de N,N-diméthyl-N'-(pyridin-3-yl)méthyl-N'-[4-(2,4,6-triisopropylphényl)thiazol-2-yl]éthane-1,2-diamine, l'acide 2-[(5-(2,6-diméthoxyphényl)-1-{4-[(3-diméthylaminopropyl) méthylcarbamoyl]-2-isopropylphényl}-1*H*-pyrazole-3-carbonyl)amino]adamantane-2-carboxylique, le 3-(1-{2-[4-benzoyl-2-(3,4-difluorophényl)morpholino-2-yl]éthyl}-4-phényl-pipéridin-4-yl)-1,1-diméthylurée, le trichlorhydrate de l'acide 3-[N-{4-[4-(aminoiminométhyl)phényl]-1,3-thiazol-2-yl}-N-(1-carboxy-méthylpipéridin-4-yl)amino] propionique, le 3-[N-{4-[4-(amino(N-éthoxycarbonyl-imino)méthyl)phényl]-1,3-thiazol-2-yl}-N-(1-(éthoxycarbonylméthyl)pipéridin-4-yl)amino]propionate d'éthyle, le 5-éthoxy-1-{4-(N-ter-butylcarbamoyl)-2-méthoxybenzènesulfonyl]-3-spiro-[4-(2-morpholinoéthyloxy)cyclohexane]indolin-2-one et leurs sels pharmaceu-tiquement acceptables

Les formulations suivantes sont particulièrement avantageuses :
- toute formulation obtenue par lyophilisation d'une solution dans laquelle le mannitol est à une concentration de 9 mg par ml, l'alanine est à une concentration de 18 mg par ml et le principe actif est l'acide 2-{[4-(2-chlorophényl)thiazol-2yl]aminocarbonyl}indol-1-acétique à une concentration de 1,18 mg par ml ou un de ses sels pharmaceutiquement acceptables à une concentration équivalente;
- toute formulation obtenue par lyophilisation d'une solution dans laquelle le mannitol est à une concentration de 10 mg par ml, l'alanine est à une concentration de 23 mg par ml et le principe actif est l'irbésartan à une concentration de 1 mg par ml ou un de ses sels pharmaceutiquement acceptables à une concentration équivalente; et
- toute formulation obtenue par lyophilisation d'une solution dans laquelle le mannitol est à une concentration de 9 mg par ml, l'alanine est à une concentration de 18 mg par ml et le principe actif est le chlorhydrate de 1-(2-naphtalèn-2-yl-éthyl)-4-(3-trifluorométhylphényl)-1,2,3,6-tétrahydropyridine à une concentration comprise entre 0,01 mg et 0,2 mg par ml ou un de ses sels pharmaceutiquement acceptables à une concentration équivalente.

Un sel pharmaceutiquement acceptable de l'un quelconque des principes actifs salifiables énumérés ci-dessus peut également être sélectionné à titre de principe actif.

Le principe actif pharmaceutique est de préférence choisi parmi le groupe constitué par le sel de potassium de l'acide SR 27897 désigné ci-après par SR 27897B, l'irbésartan ou SR 47436, le clopidogrel, l'acide ursodésoxycholique ou son sel de sodium, le SR 57746A et le SR 27417A.

Afin d'illustrer la présente invention, sans toutefois la limiter, des évaluations ont été effectuées en choisissant comme exemple de principe actif pharmaceutique le SR 27897B, le SR 47436 (l'irbésartan) et le SR 57746A. Ainsi plusieurs solutions contenant le SR 27897B à 1 mg/ml, un tampon phosphate (Na₂HPO₄ /NaH₂PO₄) à différentes molarités et à des pH compris entre 7,5 et 8,25; du mannitol et de l'alanine dans un rapport R = masse de mannitol / masse d'alanine compris entre 0,1 et 1 ont été préparées lyophilisées et analysées.

De même, plusieurs solutions contenant le SR 47436 à 1 mg/ml, de l'hydroxyde de potassium dans un rapport molaire [KOH] / [SR 47436] supérieur ou égal à 1, de l'alanine seule ou un mélange mannitol-alanine dans un rapport R = masse de mannitol / masse d'alanine compris entre 0,1 et 1, et de l'éthanol, ont été préparées lyophilisées et analysées.

Enfin, une solution contenant le SR 57746A sous forme de chlorhydrate à 0,11 mg/ml, de l'acide citrique anhydre et un mélange mannitol-alanine dans un rapport R = masse de mannitol/masse d'alanine égal à 0,5 a été préparée lyophilisée et analysée.

Le TABLEAU 1 ci-après indique les compositions des solutions étudiées contenant le SR 27897B. Pour chacune de ces formulations R = 0,5 avec comme concentration de mannitol, d'alanine et de SR 27897B respectivement 9 mg/ml, 18 mg/ml et 1 mg/ml.

**TABLEAU 1**

| **LOT N°** | **TAMPON PHOSPHATE DE SODIUM : mM** | **pH DU TAMPON PHOSPHATE DE SODIUM** |
|---|---|---|
| 1 | 5 | 7,5 |
| 2 | 5 | 8 |
| 3 | 10 | 8 |
| 4 | 25 | 8 |
| 5 | 25 | 8,5 |
| 6 | 15 | 8 |
| 7 | 25 | 7,75 |
| 8 | 25 | 8 |
| 9 | 25 | 8,25 |
| 10 | 35 | 8,25 |
| 11 | 25 | 8 |

Le TABLEAU 2 ci-dessous indique la composition des solutions lyophilisées étudiées contenant du SR 47436.

**TABLEAU 2**

| **LOT N°** | **mg/ml MANNITOL** | **mg/ml ALANINE** | **R** | **mg/ml KOH** | **mg/ml SR 47436** |
|---|---|---|---|---|---|
| 12 | 10 | 18 | 0,55 | 0,137 | 1 |
| 13 | 10 | 23 | 0,43 | 0,137 | 1 |

Le TABLEAU 3 ci-dessous indique la composition des solutions lyophilisées étudiées contenant le SR 57746A sous forme de chlorhydrate.

**TABLEAU 3**

| **LOT N°** | **mg/ml MANNITOL** | **mg/ml ALANINE** | **R** | **mg/ml ACIDE CITRIQUE ANHYDRE** | **mg/ml SR 57746A** | **mg/ml POLYSORBATE 80** |
|---|---|---|---|---|---|---|
| 14 | 9 | 18 | 0,5 | 7,7 | 0,11 | 0 |
| 15 | 9 | 18 | 0,5 | 7,7 | 0,11 | 1 |

La turbidité des lyophilisats repris en solution sera déterminée à l'aide d'un turbidimètre Ratio Hach 18900-00. Les résultats seront exprimés en unités néphélométriques de turbidité (NTU) définie par *Standard methods for the examination of water and wastewater de l'American Public Health Association.*

Les critères organoleptiques des lyophilisats seront examinés visuellement et prendront en compte la coloration du lyophilisat, sa structure (effondré ou non), ainsi que l'observation d'un déphasage éventuel entre la croûte et la mie du lyophilisat.

La teneur en eau des lyophilisats sera déterminée par coulométrie selon la méthode décrite dans Ph. Fr. Xème Ed. V. 3.5.6.A., en injectant à l'aide d'une seringue 2 ml de méthanol dans le flacon du lyophilisat. La teneur en eau sera exprimée en pourcentage en poids du lyophilisat.

L'analyse diffractométrique des Rayons X sur les lyophilisats sera effectuée sur un diffractomètre SIEMENS D500 TT ; Source : CuKal ; Générateur : 40 KV, 25 mA ; Monochromateur arrière ; Fentes : 1/1/1/0,16/0,6 ; Echantillonnage sur portoir pyrex ; Domaine de balayage : 4° à 40° par minute en 2 thêta de Bragg.

L'analyse thermique différentielle (DSC) sera effectuée en utilisant l'appareil DSC 7 Perkin Elmer avec les caractéristiques suivantes : étalonnage à l'indium et au plomb, prise d'essai entre 5 et 10 mg dans une capsule de 50 µl, température initiale de 10°C, vitesse de chauffage de 10°C/minute, température finale de 300°C.

Le dosage du SR 27897B sera effectué par chromatographie liquide (Ph. Eur. 2. (I) V. 6.20.4.) à 254 nm en utilisant une colonne greffée C18 de 25 cm de longueur, de 4,6 mm de diamètre interne et de granulométrie 10 µm (Bischoff référence 25461840). La phase mobile sera constituée par un mélange volume à volume de tampon acétate pH 4,0 (acide acétique glacial et ammoniaque concentrée Merck) et d'acétonitrile pour chromatographie (Sharlau référence Ac33). La solution témoin sera constituée d'une solution de SR 27897B (foumi par Sanofi Recherche) à 50 µg par ml de méthanol (Merck référence 6009). La solution à analyser sera obtenue par dissolution du lyophilisat dans 100 ml d'eau ultra purifiée (Millipore, eau "Milli-Q"). Le débit sera de 2 ml/mn. On calculera la surface des pics spécifiques obtenue après injection du 20 µl de solution témoin puis de solution à analyser pour chacun des chromatogrammes. La teneur en SR 27897B du lyophilisat exprimée en mg/flacon pourra être déterminée à partir du calcul de ces deux surfaces.

Le dosage des substances apparentées (impuretés) du SR 27897B dans le lyophilisat en cours de conservation, paramètre significatif de la stabilité du produit, sera également effectué par chromatographie liquide sur colonne greffée C18 (Bischoff référence 25461840). La phase mobile sera constituée par un gradient d'acétonitrile et de tampon acétate pH 4,0 dont la composition est indiquée dans le TABLEAU A:

**TABLEAU A**

| **TEMPS (minute)** | **ACETONITRILE (volume)** | **TAMPON ACETATE pH 4,0 (volume)** |
|---|---|---|
| 0 | 20 | 80 |
| 5 | 30 | 70 |
| 15 | 60 | 40 |
| 25 | 70 | 30 |
| 28 | 20 | 80 |
| 40 | 20 | 80 |

La solution témoin sera constituée d'une solution de SR 27897B (Sanofi Recherche) à 10 µg par ml de méthanol. La solution à analyser sera obtenue par dissolution du contenu d'un flacon lyophilisé dans 5 ml de méthanol. Le débit sera de 2 ml/mn. On calculera de la même manière la surface des pics spécifiques des impuretés inconnues obtenue sur les chromatogrammes après injection de 20 µl de la solution à analyser, rapportée à la surface du pic spécifique de SR 27897B obtenue après injection de 20 µl de la solution témoin. La teneur en chacune des impuretés inconnues et la teneur globale en impuretés du SR 27897B lyophilisé, exprimées en pourcentage en poids du produit, pourront être déterminées à partir de ces calculs.

Le dosage du SR 47436 sera effectué par chromatographie liquide HPLC ( Ph. Eur. 2 (I) V 6.20.4.) à 220 nm en utilisant une colonne de silice greffée C18 en acier inoxydable, de 25 cm de longueur, 8 mm de diamètre exteme et 4 mm de diamètre inteme, silice sphérique de diamètre 7 µM et de 120 Å de diamètre de pores ayant subi un traitement d"'end capping" (colonne référence 720042 foumie par Chromoptic). La phase mobile sera constituée par un mélange de 60 volumes de solution de tampon phosphate pH 3,0 (acide phosphorique Prolabo référence 20624295, triéthylamine Fluka référence 90340) et de 40 volumes d'acétonitrile pour chromatographie (Merck référence 14291) avec, un débit de 1 ml/mn.

La première solution témoin sera constituée par une solution de SR 47436 (Sanofi Recherche) à 0,5 mg par ml de phase mobile. La seconde solution témoin sera constituée par une solution contenant 0,5 mg de SR 47436 et 0,5 mg d'impureté correspondant au produit d'ouverture (Sanofi Recherche) par ml de phase mobile. La solution à analyser sera obtenue par dissolution du lyophilisat dans 10 ml de phase mobile. On s'assurera par injection successive de la première et seconde solution témoin que les conditions opératoires sont satisfaisantes (facteur de résolution supérieur à 2 entre les deux pics pour une injection de 10 µl de la seconde solution témoin, coefficient de variation de la surface du pic inférieur ou égal à 1 % pour une série de 5 injections de 10 µl de la première solution témoin). Après injection de 10 µl de chaque solution témoin et de 20 µl de chaque solution à analyser, on déterminera par le calcul des surfaces des pics spécifiques obtenues sur les chromatogrammes la teneur en SR 47436 en mg par lyophilisât.

Le dosage des substances apparentées (impuretés) de SR 47436 sera effectué par chromatographie liquide HPLC ( Ph. Eur. 2 (I) V 6.20.4.) à 220 nm en utilisant une colonne C18 de silice greffée (cf dosage de SR 47436). La phase mobile sera constituée par un mélange de 60 volumes de tampon phosphate pH 3,1 et de 40 volumes d'acétonitrile pour chromatographie avec un débit de 1 ml/mn. Les deux solutions témoins seront constituées pour la première par une solution de SR 47436 (Sanofi Recherche) à 0,5 mg par ml de méthanol (foumi par SDS sous la référence 0930221) et pour la seconde par une solution de SR 47436 à 0,5 µg par ml de méthanol. La solution à analyser sera obtenue par dissolution du lyophilisat dans 10 ml d'eau pour préparations injectables (PPI). L'analyse devant être effectuée au plus tard dans la demi-heure suivant la reconstitution. On s'assurera de conditions opératoires satisfaisantes par injections successives de 10 µl d'eau pour préparations injectables et 10 µl des deux solutions témoins (temps de rétention du pic principal voisin pour les deux témoins, rapport signal sur bruit supérieur ou égal à 10 pour le premier témoin). Après injection de 10 µl de la solution à analyser, on déterminera par le calcul des surfaces des pics spécifiques obtenues sur les chromatogrammes les teneurs par substance apparentée et la teneur globale en substances apparentées (impuretés) exprimées en pourcentage de poids du produit.

Le dosage du SR 57746A (Sanofi Recherche) sera effectué par chromatographie liquide à 224 nm en utilisant une colonne de silice greffée C18 de 25 cm de longueur, de 4 mm de diamètre interne et de granulométrie 7 µm (Macherey Nagel, référence 720042). La phase mobile sera constituée par un mélange de 45 volumes d'acétonitrile pour chromatographie (Rathbum référence RH 1016) et de 55 volumes de solution tampon pH 3,0 (préparée en diluant 5,5 ml d'acide phosphorique dans 950 ml d'eau déminéralisée filtrée (Millipore Alpha-Q), puis en ajustant à pH 3,0 avec une solution de triéthylamine (Fluka, référence 90340), en ajoutant ensuite 10 ml d'acétonitrile et en complétant à 1000 ml avec de l'eau déminéralisée filtrée). La solution témoin sera constituée d'une solution de SR 57746A à 15,0 mg par 100 ml de méthanol (Carlo Erba, référence 414814). La solution à analyser sera obtenue par dissolution du lyophilisat dans 3,0 ml d'un mélange constitué de 25 volumes de méthanol et de 75 volumes d'eau déminéralisée filtrée. Le débit sera de 1 ml par minute. On mesurera la surface des pics spécifiques obtenus après injection de 10 µl de solution témoin puis de solution à analyser pour chacun des chromatogrammes. La teneur en SR 57746A du lyophilisat, exprimée en mg/flacon pourra être déterminée à partir de la mesure des deux surfaces.

Le dosage des substances apparentées (impuretés) du SR 57746A dans le lyophilisat en cours de conservation sera également effectuée par chromatographie liquide sur colonne avec les conditions chromatographiques décrites dans « Dosages » de (Ph. Eur. 2. (I) V.6.20.4). La solution témoin sera constituée d'une solution de SR 57746A à 0,15 µg par ml de méthanol. La solution à analyser sera obtenue par dissolution du contenu du lyophilisat dans 3 ml d'un mélange de 25 volumes de méthanol et de 75 volumes d'eau déminéralisée filtrée. Le débit sera de 1 ml par minute. On mesurera de la même manière la surface des pics spécifiques des impuretés inconnues obtenues sur les chromatogrammes après injection de 10 µl de la solution à analyser, rapportée à la surface du pic spécifique de SR 57746A obtenue après injection de 10 µl de solution témoin. La teneur en chacune des impuretés inconnues et la teneur globale en impuretés du SR 57746A lyophilisé, exprimées en pourcentage en surface, pourront être déterminées à partir de ces mesures.

Les résultats analytiques obtenus en utilisant ces différentes méthodes sont décrits ci-après.

Le TABLEAU 4 ci-après représente les résultats des contrôles initiaux effectués sur les lyophilisats de SR 27897B pour la teneur en eau (en % en poids du lyophilisât), la température de transition vitreuse Tg (en °C) déterminée par DSC, et sur les lyophilisats repris par de l'eau PPI pour la turbidité (en NTU) et le pH.

**TABLEAU 4**

| Lot N° | % Eau | Tg °C | Turbidité NTU | pH |
|---|---|---|---|---|
| 1 | 0,39 | 27 | 58 | 7,25 |
| 2 | 0,47 | 25 | 23 | 7,2 |
| 3 | 0,6 | 27 | 11 | 7,4 |
| 4 | 0,91 | 35 | 2,2 | 7,6 |
| 5 | 0,89 | 40 | 29 | 7,5 |
| 6 | 0,61 | 36,7 | 17 | 7,6 |
| 7 | 0,93 | 45,5 | 33 | 7,6 |
| 8 | 1,07 | 46,1 | 1,4 | 7,8 |
| 9 | 1,04 | 45,7 | 0,5 | 7,7 |
| 10 | 1,94 | 45,8 | 0,8 | 7,8 |
| 11 | | | 4,5 | 7,6 |

A titre d'exemple supplémentaire plusieurs lots de lyophilisats de SR 27897B ont été suivis en stabilité à 5°C, 25°C, 40°C et 50°C pendant 1 mois, 3 mois et 6 mois.

Le TABLEAU 5 qui représente la teneur totale en substances apparentées (impuretés), exprimée en % en poids de SR 27897B initial, retrouvées dans les lyophilisats de SR 27897B après 1 mois de conservation montre que la stabilité est excellente après ce temps de conservation.

**TABLEAU 5**

| Lot n° | % impuretés à 5°C | % impuretés à 25°C | % impuretés à 40°C | % impuretés à 50°C |
|---|---|---|---|---|
| 1 | <0,27 | <0,26 | | <0,25 |
| 2 | <0,23 | <0,26 | | <0,24 |
| 3 | <0,23 | <0,26 | | <0,25 |
| 4 | <0,26 | <0,24 | | < 0,24 |
| 5 | < 0,23 | < 0,25 | | <0,26 |
| 6 | | | <0,1 | |
| 7 | | | <0,1 | |
| 8 | | | <0,1 | |
| 9 | | | <0,1 | |
| 10 | | | < 0,1 | |
| 11 | | <0,1 | <0,1 | |

Le TABLEAU 6 qui représente la teneur totale en substances apparentées (impuretés) retrouvées dans les lyophilisats de SR 27897B après 3 mois de conservation à 50°C, montre que la stabilité est excellente après ce temps de conservation.

**TABLEAU 6**

| Lot n° | % impuretés à 50°C |
|---|---|
| 1 | <0,1 |
| 2 | <0,1 |
| 3 | <0,1 |
| 4 | <0,1 |
| 5 | <0,1 |

Enfin le TABLEAU 7 représentant la teneur totale en substances apparentées (impuretés) retrouvées dans les lyophilisats de SR 27897B après 6 mois de conservation à 5°C et 40°C, montre que la stabilité est également excellente après ce temps de conservation

**TABLEAU 7**

| Lot n° | % impuretés à 5°C | % impuretés à 40°C |
|---|---|---|
| 7 | < 0,1 | 0,13 |
| 8 | <0,1 | 0,1 |
| 9 | <0,1 | 0,1 |
| 11 | | <0,1 |

### Diffraction des rayons X.

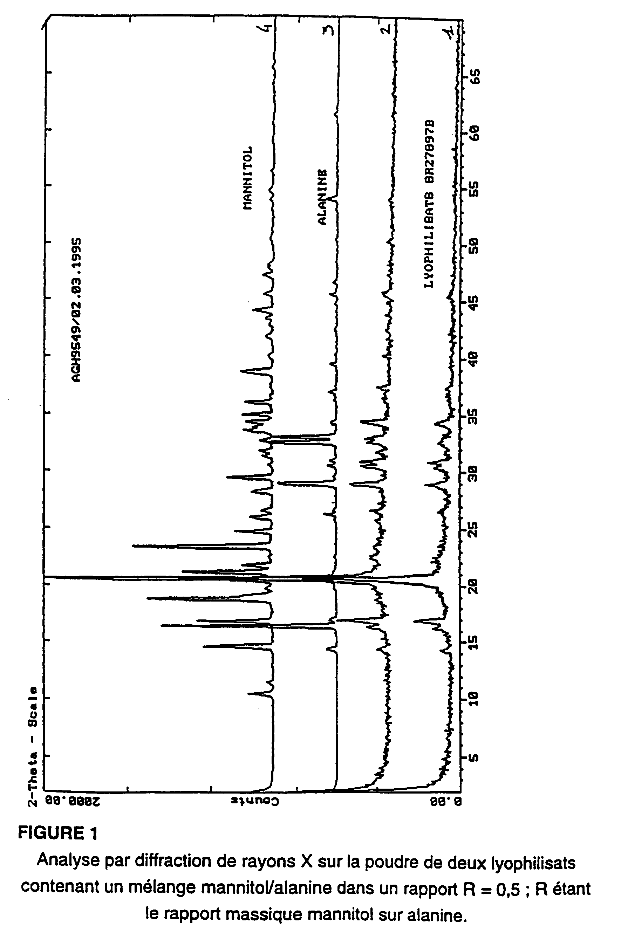

Le résultat de l'analyse par diffraction de rayons X sur la poudre de deux lyophilisats contenant un mélange mannitol/alanine dans un rapport R = masse de mannitol / masse d'alanine = 0,5 est indiqué sur la figure 1, diffractogrammes 1 et 2. Les diffractogrammes 3 et 4 de la figure 1 représentent les témoins alanine et mannitol. Comme nous pouvons l'observer sur cette figure, la raie située entre 10° et 11°, caractéristique du mannitol cristallisé, n'est pas obtenue pour les deux lyophilisats de SR 27897B. Ainsi pour R = 0,5 ; l'alanine est seule sous forme cristallisée, le mannitol étant lui sous forme amorphe.

### Analyse thermique différentielle.

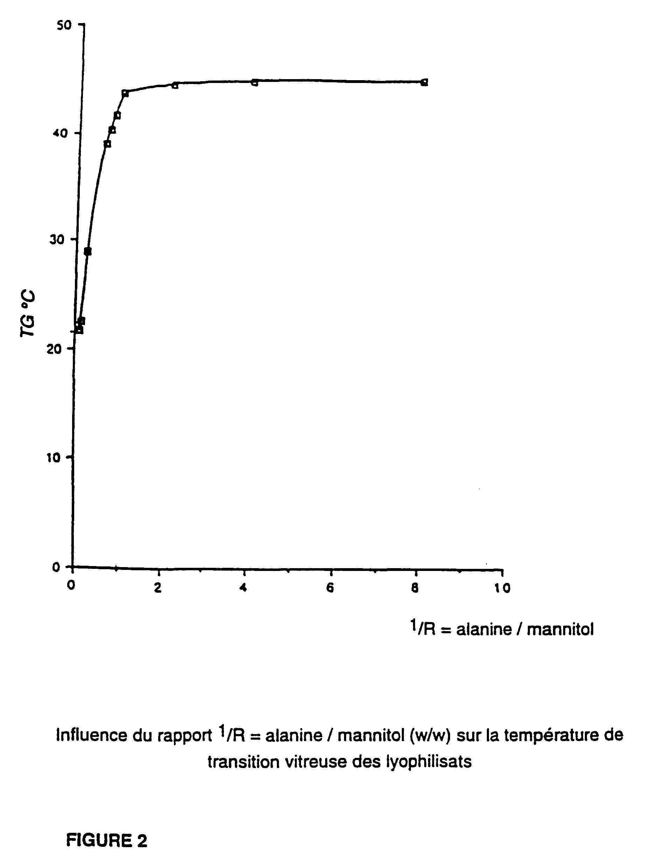

La figure 2 représente l'influence du rapport massique alanine sur mannitol sur la température de transition vitreuse des lyophilisats. Cette figure nous montre que la température de transition vitreuse maximale est obtenue pour (1/R) > 1 c'est à dire pour R compris entre 0 et 1. En général, la température de transition vitreuse est représentative de la température maximale de stabilité du lyophilisat. Ainsi la température maximale de stabilité du lyophilisat est atteinte pour R compris entre 0 et 1.

Le TABLEAU 8 ci-dessous représente les résultats des contrôles initiaux effectués sur les lyophilisats de SR 47436 pour la teneur en eau, la teneur totale en substances apparentées (impuretés) et sur les lyophilisats repris par de l'eau PPI pour le pH.

**TABLEAU 8**

| Lot n° | % eau | % impuretés | pH |
|---|---|---|---|
| 12 | 0,5% | 0,24 | 6,6 |
| 13 | 0,2 | 0,1 | 6,7 |

Le TABLEAU 9 ci-dessous représente les teneurs totales en substances apparentées exprimées en pourcentage de pureté en SR 47436 des lyophilisats de SR 47436 lot 12 après 1 semaine et 2 semaines de conservation à 5°C, 25°C, 35°C et 50°C.

**TABLEAU 9**

| Lot n°12 | 5°C | 25°C | 35°C | 50°C |
|---|---|---|---|---|
| 1 semaine | 99,89 | 99,89 | 99,71 | 98,47 |
| 2 semaines | 99,90 | 99,82 | 99,47 | 97,05 |

Le TABLEAU 10 ci-après représente les teneurs totales en substances apparentées exprimées en pourcentage d'impuretés, des lyophilisats de SR 47436 lot 13 après 3 mois, 6 mois et 9 mois de conservation à 5°C, 25°C et 35°C.

**TABLEAU 10**

| Lot n°13 | 5°C | 25°C | 35°C |
|---|---|---|---|
| 3 mois | 0,1% | 0,2% | 0,3% |
| 6 mois | 0,2% | 0,3% | 0,6% |
| 9 mois | 0,3% | 0,3% | - |

Le TABLEAU 11 ci-dessous représente les teneurs totales en substances apparentées exprimées en pourcentage d'impuretés, des lyophilisats de SR 57746A après 1 mois et 3 mois de conservation à 5°C, 25°C et 40°C et d'un lyophilisât de SR 57746A reconstitué immédiatement (référence)

**TABLEAU 11**

| Lots n°14 et n° 15 | 5°C | 25°C | 40°C |
|---|---|---|---|
| référence | | < 0,1 % | |
| 1 mois | < 0,1 % | < 0,1 % | < 0,1 % |
| 3 mois | < 0,1 % | < 0,1 % | < 0,1 % |

### EXEMPLE 1 : Composition d'un lyophilisat de SR 27897 (base) à reprendre par 1 ml d'eau PPI

| CONSTITUANTS | Formule unitaire en (mg) |
|---|---|
| SR 27897B* | 1,18 mg |
| Alanine apyrogène | 18,0 mg |
| Mannitol | 9,0 mg |
| Phosphate monosodique dihydraté apyrogène | 0,3 mg |
| Phosphate disodique dodécahydraté apyrogène | 8,5 mg |
| Flacon verre blanc type 1 de 3 ml | 1 |
| Bouchon pilier en chlorobutyle gris | 1 |
| Capsule en aluminium Flip-off bleue diamètre 13 mm | 1 |

| | |
|---|---|
| *correspondant à 1 mg en SR 27897 acide | |

### EXEMPLE 2 : Composition d'un lyophilisat de SR 27897 (base) à reprendre par 5 ml d'eau PPI

| CONSTITUANTS | Formule unitaire en (mg) |
|---|---|
| SR 27897B* | 5,9 mg |
| Alanine apyrogène | 90,0 mg |
| Mannitol apyrogène | 45,0 mg |
| Phosphate monosodique dihydraté apyrogène | 1,5 mg |
| Phosphate disodique dodécahydraté apyrogène | 42,5 mg |
| Flacon verre blanc type 1 de 20 ml | 1 |
| Bouchon pilier en chlorobutyle gris diamètre 20 mm | 1 |
| Capsule en aluminium Flip-off bleue diamètre 20 mm | 1 |

| | |
|---|---|
| *correspondant à 5 mg en SR 27897 acide | |

### EXEMPLE 3 : Composition d'un lyophilisât de SR 47436 à 5 mg à reprendre par 5 ml d'eau PPI

| CONSTITUANTS | Formule unitaire en (mg) |
|---|---|
| SR 47436 | 5,0 mg |
| Alanine apyrogène | 115,0 mg |
| Mannitol apyrogène | 50,0 mg |
| hydroxyde de potassium | 0,687 mg |
| Flacon verre blanc type 1 de 20 ml | 1 |
| Bouchon pilier en chlorobutyle gris, diamètre 20 mm | 1 |
| Capsule aluminium operculée, diamètre 20 mm | 1 |

### EXEMPLE 4 : Composition d'un lyophilisât de SR 57746A (chlorhydrate) à reprendre par 4 ml d'eau PPI

| CONSTITUANTS | Formule unitaire en (mg) |
|---|---|
| SR57746A | 0,44 mg |
| Alanine apyrogène | 72,0 mg |
| Mannitol apyrogène | 36,0 mg |
| Acide citrique anhydre apyrogène | 30,8 mg |
| Flacon verre blanc type 1 de 20 ml | 1 |
| Bouchon pilier en chlorobutyle gris diamètre 20 mm | 1 |
| Capsule en aluminium Flip-off bleue diamètre 20mm | 1 |

### EXEMPLE 5 : Composition d'une solution de SR 57746A (chlorhydrate) à lyophiliser exprimée en concentration pour des volumes finaux de solution pouvant atteindre 100 ml par addition d'une quantité d'eau PPI adéquate.

| CONSTITUANTS | Formule unitaire exprimée en mg/ml |
|---|---|
| SR57746A | 0,11 mg/ml |
| Alanine apyrogène | 18,0 mg/ml |
| Mannitol apyrogène | 9,0 mg/ml |
| Acide citrique anhydre apyrogène | 7,7 mg/ml |
| Eau pour préparations injectables | QSP 1 ml |
| Flacon verre blanc type 1 | 1 |
| Bouchon pilier en chlorobutyle gris | 1 |
| Capsule en aluminium Flip-off bleue | 1 |

### EXEMPLE 6 : Composition d'un lyophilisat de SR 57746A (chlorhydrate) contenant de 0,01 mg à 0,2 mg de SR 57746A (chlorhydrate) à reprendre par 1 ml d'eau PPI.

| CONSTITUANTS | Formule unitaire en (mg) |
|---|---|
| Alanine apyrogène | 18,0 mg |
| Mannitol apyrogène | 9,0 mg |
| Acide citrique anhydre apyrogène | 7,7 mg |
| Flacon verre blanc type 1 de 3 ml | 1 |
| Bouchon pilier en chlorobutyle gris | 1 |
| Capsule en aluminium Flip-off bleue diamètre 13mm | 1 |

### EXEMPLE 7 : Composition d'un lyophilisât de SR 57746A (chlorhydrate) à reprendre par 4 ml d'eau PPI

| CONSTITUANTS | Formule unitaire en (mg) |
|---|---|
| SR57746A | 0,44 mg |
| Alanine apyrogène | 72,0 mg |
| Mannitol apyrogène | 36,0 mg |
| Acide citrique anhydre apyrogène | 30,8 mg |
| Polysorbate 80 | 4,0 mg |
| Flacon verre blanc type 1 de 20 ml | 1 |
| Bouchon pilier en chlorobutyle gris diamètre 20 mm | 1 |
| Capsule en aluminium Fiip-off bleue diamètre 20 mm | 1 |

### EXEMPLE 8 : Composition d'une solution de SR 57746A (chlorhydrate) à lyophiliser expriméé en concentration pour des volumes finaux de solution pouvant atteindre 100 ml par addition d'une quantité d'eau PPI adéquate.

| CONSTITUANTS | Formule unitaire exprimée en mg/ml |
|---|---|
| SR57746A | 0,11 mg/ml |
| Alanine apyrogène | 18,0 mg/ml |
| Mannitol apyrogène | 9,0 mg/ml |
| Acide citrique anhydre apyrogène | 7,7 mg/ml |
| Polysorbate 80 | 1,0 mg/ml |
| Eau pour préparations injectables | QSP 1 ml |
| Flacon verre blanc type 1 | 1 |
| Bouchon pilier en chlorobutyle gris | 1 |
| Capsule en aluminium Flip-off bleue | 1 |

### EXEMPLE 9 : Composition d'un lyophilisat de SR 57746A (chlorhydrate) contenant de 0,01 mg à 0,2 mg de SR 57746A (chlorhydrate) à reprendre par 1 ml d'eau PPI.

| CONSTITUANTS | Formule unitaire en (mg) |
|---|---|
| Alanine apyrogène | 18,0 mg |
| Mannitol apyrogène | 9,0 mg |
| Acide citrique anhydre apyrogène | 7,7 mg |
| Polysorbate 80 | 1,0 mg |
| Flacon verre blanc type 1 de 3 ml | 1 |
| Bouchon pilier en chlorobutyle gris | 1 |
| Capsule en aluminium Flip-off bleue diamètre 13 mm | 1 |

## Revendications

1. Formulation lyophilisée constituée d'une phase amorphe et d'une phase cristalline, pharmaceutiquement acceptable comprenant au moins un principe actif non protéique, **caractérisée en ce qu'**elle contient du mannitol et de l'alanine dans un rapport R compris entre 0,1 et 1, R représentant la masse de mannitol sur la masse d'alanine, les formulations comprenant en outre un ou plusieurs agents formant matrice choisis dans le groupe suivant étant exclues : les dérivés protéiques d'origine animale ou végétale tels que les gélatines, les dextrines ou les protéines extraites de graines de blé ou de soja, les gommes telles que l'agar ou le xanthane, les polysaccharides, les alginates, les carboxyméthylcelluloses, les pectines, les polymères synthétiques comme la polyvinylpyrrolidone, les complexes de nature polysaccharidique comme la gélatine d'acacia et leurs mélanges.

2. Formulation selon la revendication 1 dans laquelle le principe actif est en association avec un autre principe actif de nature protéique.

3. Formulation selon la revendication 1 ou 2 comprenant en outre au moins un composé additionnel choisi parmi : un tampon, un tensio-actif, un conservateur, un sel, un antioxydant et un agent chélatant.

4. Formulation selon la revendication 1 ou 2 pour la reconstitution d'une solution pour son administration par voie parentérale.

5. Formulation selon la revendication 1 ou 2 pour la reconstitution d'une solution pour son administration par voie orale.

6. Formulation selon la revendication 4 pour la reconstitution d'une solution injectable .

7. Formulation selon la revendication 1 directement administrable par voie orale.

8. Formulation selon la revendication 1 dans laquelle le principe actif est choisi parmi le groupe constitué par les acides phénylalcanoïques, les anti-inflammatoires non stéroidiens du type oxicam, le paracétamol, l'acétylsalicylate de lysine ou d'arginine, les acides biliaires, les corticostéroïdes, les anthracyclines, le phloroglucinol, les dérivés de platine, les dérivés des alcaloïdes de la vinca minor, les dérivés des alcaloïdes de l'ergot de seigle, les dérivés des bases puriques ou pyrimidiques, les prostaglandines, les benzodiazépines, les antibiotiques bêta-lactamiques, les antibiotiques macrolides, les antibiotiques de la famille des tétracyclines, les antibiotiques du type chloramphénicol, les antibiotiques du type spiramycine, les nitroso-urées, les moutardes azotées, les H₂-antagonistes, l'oméprazole, les vitamines, les antitumoraux, les médicaments cardiovasculaires, les médicaments hématologiques, les médicaments anticoagulants et antithrombotiques, les héparinoïdes, l'oxoglutarate de diarginine, les extraits de plantes, les nucléotides, les analogues de l'acide valproïque, la métopimazine, la moxisylite, les bisphosphonates actifs en tant qu'agent antiostéoporotique, la pralidoxime, la déféroxamine, les barbituriques, le clométhiazole, les antagonistes 5-HT₂, les antagonistes de l'angiotensine II, la fantofarone, la tirapazamine, le (2S)-1-[(2R,3S) 5-chloro-3-(2-chlorophényl)-1-(3,4-diméthoxybenzènesulfonyl)-3-hydroxy-2,3-dihydro-1*H*-indole-2-carbonyl]-pyrrolidine-2-carboxamide, le N,N-dibutyl-3-{4-[(2-butyl-5-méthylsulfonamido)-benzofuran-3-yl-carbonyl]phénoxyl}propylamine, le 6-(2-diéthylamino-2-méthyl)propylamino-3-phényl-4-propylpyridazine, l'éthyl{(7S)-7-[(2R)-2-(3-chlorophényl)-2-hydroxyéthylamino]-5,6,7,8-tétrahydro-naphthalèn-2-yloxy}-acétate, le 1-(2,4-dichlorophényl)-3-(N-pipéridin-1-yl-carboxamido)-4-méthyl-5-(4-chlorophényl)-1*H*-pyrazole, le 4-{[N-(3,4-diméthoxy-phénéthyl)]-N-méthylaminopropoxyl}-2-benzènesulfonyl-3-isopropyl-1-méthylindole, l'acide 2-{[1-(7-chloroquinolin-4-yl)-5-(2,6-diméthoxyphényl)-1*H*-pyrazole -3-carbonyl]amino} adamantane-2-carboxylique, le N-cyclohexyl-N-éthyl-3-(3-chloro-4-cyclohexylphényl)prop-2-énylamine,le (-)-N-méthyl-N-[4-(4-acétylamino-4-phénylpipéridino)-2-(3,4-dichlorophényl)butyl]benzamide, le chlorure de (S)-1-{2-[3-(3,4-dichlorophényl)-1-(3-isopropoxyphénylacétyl)-pipéridin-3-yl]éthyl}-4-phényl-1-azoniabicyclo[2.2.2] octane et ses sels quaternaires pharma-ceutiquement acceptables, le 4-amino-1-(6-chloropyrid-2-yl)pipéridine, le (S)-N-(1-{3-[1-benzoyl-3-(3,4-dichlorophényl)pipéridin-3-yl]propyl}-4-phénylpipéridin-4-yl)-N-méthylacétamide, l'acide 2-{[4-(2-chlorophényl)thiazol-2-yl]aminocarbonyl}indole-1-acétique, le clopidogrel, le chlorhydrate de 1-(2-naphtalèn-2-yléthyl)-4-(3-trifluorométhylphényl)-1,2,3,6-tétrahydropyridine, la N,N-diméthyl-N'-(pyridin-3-yl)méthyl-N'-[4-(2,4,6-triisopropylphényl)thiazol-2-yl]éthane-1,2-diamine, et leurs sels pharmaceutiquement acceptables.

9. Formulation selon la revendication 1 dans laquelle le principe actif est choisi parmi l'acide 2-{[4-(2-chlorophényl)thiazol-2-yl]aminocarbonyl}indole-1-acétique ou son sel de potassium, l'irbésartan, le clopidogrel, l'acide ursodésoxycholique et son sel de sodium, le chlorhydrate de 1-(2-naphtalèn-2-yléthyl)-4-(3-trifluorométhylphényl)-1,2,3,6-tétrahydropyridine, le fumarate de N,N-diméthyl-N'-(pyridin-3-yl)méthyl-N'-[4-(2,4,6-triisopropylphényl)thiazol-2-yl]éthane-1,2-diamine, l'acide 2-[(5-(2,6-diméthoxyphényl)-1-{4-[(3-diméthylaminopropyl)méthylcarbamoyl]-2-isopropyl-phényl}-1*H*-pyrazole-3-carbonyl)amino]adamantane-2-carboxylique, le 3-(1-{2-[4-benzoyl-2-(3,4-difluorophényl)morpholino-2-yl]éthyl}-4-phényl-pipéridin-4-yl)-1,1-diméthylurée, le trichlorhydrate de l'acide 3-[N-{4-[4-(aminoiminométhyl)phényl]-1,3-thiazol-2-yl}-N-(1-carboxyméthylpipéridin-4-yl)amino]propionique, le 3-[N-{4-[4-(amino(N-éthoxycarbonylimino)méthyl)phényl]-1,3-thiazol-2-yl}-N-(1-(éthoxycarbonylméthyl)pipéridin-4-yl)amino]propionate d'éthyle, le 5-éthoxy-1-[4-(N-*ter*-butylcarbamoyl)-2-méthoxy-benzènesulfonyl]-3-spiro-[4-(2-morpholinoéthyloxy)-cyclohexane]indolin-2-one et leurs sels pharmaceutiquement acceptables.

10. Formulation selon la revendication 1 obtenue après lyophilisation d'une solution dans laquelle le mannitol est à une concentration de 9 mg par ml, l'alanine est à une concentration de 18 mg par ml et le principe actif est l'acide 2-{[4-(2-chlorophényl)thiazol-2yl]aminocarbonyl}indol-1-acétique à une concentration de 1,18 mg par ml ou un de ses sels pharmaceutiquement acceptables à une concentration équivalente

11. Formulation selon la revendication 1 obtenue après lyophilisation d'une solution dans laquelle le mannitol est à une concentration de 10 mg par ml, l'alanine est à une concentration de 23 mg par ml et le principe actif est l'irbésartan à une concentration de 1 mg par ml ou un de ses sels pharmaceutiquement acceptables à une concentration équivalente.

12. Formulation selon la revendication 1 obtenue après lyophilisation d'une solution dans laquelle le mannitol est à une concentration de 9 mg par ml, l'alanine est à une concentration de 18 mg par ml et le principe actif est le chlorhydrate de 1-(2-naphtalèn-2-yl-éthyl)-4-(3-trifluorométhylphényl)-1,2,3,6-tétrahydropyridine à une concentration comprise entre 0,01 mg et 0,2 mg par ml ou un de ses sels pharmaceutiquement acceptables à une concentration équivalente.

13. Procédé pour la stabilisation d'un principe actif non protéique contenu dans une formulation lyophilisée pharmaceutiquement acceptable, comprenant les étapes consistant à :
- dissoudre dans de l'eau pour préparation injectable ou dans un agent solubilisant, à la température de dissolution appropriée, le principe actif non protéique, du mannitol et de l'alanine dans un rapport R (mannitol/alanine) compris entre 0,1 et 1, le cas échéant en présence d'un tampon et de conservateurs ;
- filtrer les solutions obtenues ; et
- procéder à la lyophilisation des solutions résultantes par mise en oeuvre d'un cycle de congélation, puis de sublimation et de dessication.

## Patentansprüche

1. Gefriergetrocknete Formulierung gebildet aus einer amorphen Phase und einer kristallinen Phase, die pharmazeutisch annehmbar ist und mindestens einen Nicht-Protein-Wirkstoff enthält, **dadurch gekennzeichnet, daß** sie Mannitol und Alanin in einem Verhältnis R zwischen 0, 1 und 1 enthält, worin R für das Verhältnis der Masse Mannitol zu der Masse Alanin steht, wobei Formulierungen ausgeschlossen sind, die zusätzlich ein oder mehrere Matrix-bildende Bestandteile enthalten, ausgewählt aus der folgenden Gruppe: Protein-Derivate tierischen oder pflanzlichen Ursprungs, wie Gelatinen, Dextrine oder aus Getreide oder Soja extrahierte Proteine, Gummen, wie Agar oder Xanthan, Polysaccharide, Alginate, Carboxymethylcellulose, Pektine, synthetische Polymere, wie Polyvinylpyrrolidon, Komplexe mit Polysaccharid-Natur, wie Acacia-Gelatine und Mischungen davon.

2. Formulierung nach Anspruch 1, worin der Wirkstoff in Kombination mit einem anderen Wirkstoff mit Protein-Natur vorliegt.

3. Formulierung nach Anspruch 1 oder 2, enthaltend zusätzlich mindestens eine zusätzliche Verbindung ausgewählt aus: einem Puffer, einem oberflächenaktiven Mittel, einem Konservierungsmittel, einem Salz, einem Antioxidans und einem Chelatbildner.

4. Formulierung nach Anspruch 1 oder 2 für die Erzeugung einer Lösung zur Verabreichung auf parenteralem Wege.

5. Formulierung nach Anspruch 1 oder 2 für die Erzeugung einer Lösung zur Verabreichung auf oralem Wege.

6. Formulierung nach Anspruch 4 für die Erzeugung einer injizierbaren Lösung.

7. Formulierung nach Anspruch 1, die direkt auf oralem Wege verabreichbar ist.

8. Formulierung nach Anspruch 1, worin der Wirkstoff ausgewählt ist aus der Gruppe, die Phenylalkansäuren, nicht-steroidale antiinflammatorische Mittel vom Typ Oxicam, Paracetamol, Lysin- oder Arginin-acetylsalicylat, Gallensäuren, Corticosteroide, Anthracyline, Fhloroglucin, Platin-Derivaten, Derivate von Vincaminor-Alkaloiden, Derivate von Mutterkorn-Alkaloiden, Derivate von Purinbasen oder Pyrrimidinbasen, Prostaglandine, Benzodiazepine, Beta-Lactam-Antibiotika, Macrolid-Antibiotika, Antibiotika der Familie der Tetracycline, Antibiotika vom Typ Chloramphenicol, Antibiotika vom Typ Spiramycin, Nitroso-Harnstoffe, azotiertem Senf, H₂-Antagonisten, Omeprazol, Vitamine, antitumorale Mittel, cardiovaskuläre Arzneimittel, hämatologische Arzneimittel, anticoagulierende und antithrombotische Arzneimittel, Heparinoide, Diargin-Oxoglutarat, Pflanzenextrakte, Nucleotide, Valproinsäure-Analoga, Metopimazin, Moxisylit, als antiosteoporotisches Mittel wirksame Diphosphonate, Pralidoxim, Deferoxamin, Barbiturate, Clomethiazol, 5-HT₂-Antagonisten, Angiotensin II-Antagonisten, Fantofaron, Tirapazamin, (2S)-1-[(2R,3S)-5-Chlor-3-(2-chlorphenyl)-1-(3,4-dimethoxybenzolsulfonyl)-3-hydroxy-2,3-dihydro-1*H*-indol-2-carbonyl]-pyrrolidin-2-carboxamid, N,N-Dibutyl-3-{4-[(2-butyl-5-methylsulfonamido)-benzofuran-3-yl-carbonyl]-phenoxy}-propylamin, 6-(2-Diethylamino-2-methyl)-propylamino-3-phenyl-4-propylpyridazin, {(7S)-7-[(2R)-2-(3-Chlorphenyl)-2-hydroxyethylamino]-5,6,7,8-tetrahydro-naphthalin-2-yloxy}-essigsäureethylester, 1-(2,4-Dichlorphenyl)-3-(N-piperidin-1-yl-carboxamido)-4-methyl-5-(4-chlorphenyl)- 1*H*-pyrazol, 4-{[N-(3,4-Dimethoxy-phenethyl)]-N-methylaminopropoxyl}-2-benzolsulfonyl-3-isopropyl-1-methylindol, 2-{[1-(7-Chlorchinolin-4-yl)-5-(2,6-dimethoxyphenyl)-1*H*-pyrazol-3-carbonyl]-amino}-adamantan-2-carbonsäure, N-Cyclohexyl-N-ethyl-3-(3-chlor-4-cyclohexylphenyl)-prop-2-enylamin, (-)-N-Methyl-N-[4-(4-acetylamino-4-phenylpiperidino)-2-(3,4-dichlorphenyl)-butyl]-benzamid, (S)-1-{2-[3-(3,4-Dichlorphenyl)-1-(3-isopropoxyphenylacetyl)-piperidin-3-yl]-ethyl}-4-phenyl-1-azoniabicyclo[2.2.2]-octan und dessen pharmazeutisch annehmbare quaternäre Salze, 4-Amino-1-(6-chlorpyrid-2-yl)-piperidin, (S)-N-(1-{3-[1-Benzoyl-3-(3,4-dichlorphenyl)-piperidin-3-yl]-propyl}-4-phenylpiperidin-4-yl)-N-methylacetamid, 2-{[4-(2-Chlorphenyl)-thiazol-2-yl]-aminocarbonyl}-indol-1-essigsäure, Clopidogrel, 1-(2-Naphthalin-2-ylethyl)-4-(3-trifluormethylphenyl)-1,2,3,6-tetrahydropyridin-Hydrochlorid, N,N-Dimethyl-N'-(pyridin-3-yl)-methyl-N'-[4-(2,4,6-triisopropylphenyl)-thiazol-2-yl)-ethan-1,2-diamin und deren pharmazeutisch annehmbare Salze umfaßt.

9. Formulierung nach Anspruch 1, worin der Wirkstoff ausgewählt ist aus 2-{[4-(2-Chlorphenyl)-thiazol-2-yl]-aminocarbonyl}-indol-1-essigsäure oder deren Kaliumsalz, Irbesatan, Clopidogrel, Ursodesoxycholinsäure und deren Natriumsalz, 1-(2-Naphthalin-2-ylethyl)-4-(3-trifluormethylphenyl)-1,2,3,6-tetrahydropyridin-Hydrochlorid, N,N-Dimethyl-N'-(pyridin-3-yl)-methyl-N'-[4-(2,4,6-trtisopropylphenyl)-thiazol-2-yl]-ethan-1,2-diamin-Fumarat, 2-[(5-(2,6-Dimethoxyphenyl)-1-{4-[(3-dimethylaminopropyl)-methylcarbamoyl]-2-isopropyl-phenyl)-1*H*-pyrazol-3-carbonyl)-amino]-adamantan-2-carbonsäure, 3-(1-{2-[4-Benzoyl-2-(3,4-difluorphenyl)-morpholino-2-yl]-ethyl}-4-phenyl-piperidin-4-yl)-1,1-dimethyl-harnstoff, 3-[N-{4-[4-(Aminoiminomethyl)-phenyl]-1,3-thiazol-2-yl}-N-( 1-carboxymethylpiperidin-4-yl)-amino-1-propionsäure-Trihydrochlorid. 3-[N-{4-[4-(Amino-(N-ethoxycarbonylimino)-methyl)-phenyl]-1,3-thiazol-2-yl}-N-(1-(ethoxycarbonylmethyl)-piperidin-4-yl)-amino]-propionsäurethylester, 5-Ethoxy-1-[4-(N-tert.-butylcarbamoyl)-2-methoxy-benzolsulfonyl]-3-spiro-[4-(2-morpholinoethyloxy)-cyclohexan]-indolin-2-on und deren pharmazeutisch annehmbaren Salzen.

10. Formulierung nach Anspruch 1 erhalten nach dem Gefriertrocknen einer Lösung, enthaltend Mannitol in einer Konzentration von 9 mg pro ml, Alanin in einer Konzentration von 18 mg pro ml und als Wirkstoff 2-{[4-(2-Chlorphenyl)-thiazol-2-yl]-aminocarbonyl}-indol-1-essigsäure in einer Konzentration von 1,18 mg pro ml oder eines ihrer pharmazeutisch annehmbaren Salze in einer äquivalenten Konzentration.

11. Formulierung nach Anspruch 1 erhalten nach dem Gefriertrocknen einer Lösung enthaltend Mannitol in einer Konzentration von 10 mg pro ml, Alanin in einer Konzentration von 23 mg pro ml und als Wirkstoff Irbesartan in einer Konzentration von 1 mg pro ml oder eines seiner pharmazeutisch annehmbaren Salze in einer äquivalenter Konzentration.

12. Formulierung nach Anspruch 1 erhalten nach dem Gefriertrockenen einer Lösung, enthaltend Mannitol in einer Konzentration von 9 mg pro ml, Alanin in einer Konzentration von 18 mg pro ml und als Wirkstoff 1-(2-Naphthalin-2-yl-ethyl)-4-(3-trifluormethylphenyl)-1,2,3,6-tetrahydropyridin-Hydrochlorid in einer Konzentration zwischen 0,01 mg und 0,2 mg pro ml oder eines seiner pharmazeutisch annehmbaren Salze in einer äquivalenten Konzentration.

13. Verfahren zur Stabilisierung eines Nicht-Protein-Wirkstoffs in einer pharmazeutisch annehmbaren gefriergetrochneten Formulierung, umfassend die folgenden Stufen:
- Lösen des Nicht-Protein-Wirkstoffs, von Mannitol und Alanin in einem Verhältnis R (Mannitol/Alanin) zwischen 0,1 und 1, gegebenenfalls in Gegenwart eines Puffers und von Konservierungsstoffen in Wasser für die Herstellung injizierbarer Präparate oder in einem löslich machenden Mittel bei einer geeigneten Lösungstemperatur;
- Filtrieren der erhaltenen Lösungen; und
- Gefriertrocknen der erhaltenen Lösungen durch einen Zyklus des Einfrierens und dann der Sublimation und der Trocknung.

## Claims

1. Pharmaceutically acceptable lyophilised formulation consisting of an amorphous phase and a crystalline phase, comprising at least one non-protein active substance, **characterised in that** it contains mannitol and alanine in a ratio R of between 0.1 and 1, R representing the mass of mannitol to the mass of alanine, formulations which further contain one or more matrix-forming agents selected from the following group being excluded: protein derivatives of animal or plant origin such as gelatins, dextrins or proteins extracted from wheat or soya grains, gums such as agar or xanthan, polysaccharides, alginates, carboxymethylcelluloses, pectins, synthetic polymers such as polyvinylpyrrolidone, complexes of a polysaccharide nature such as acacia gelatin and mixtures thereof.

2. Formulation according to claim 1, wherein the active substance is combined with another protein-type active substance.

3. Formulation according to claim 1 or 2, further comprising at least one additional compound selected from among: a buffer, a surfactant, a preservative, a salt, an antioxidant and a chelating agent

4. Formulation according to claim 1 or 2, for reconstituting a solution for administration by parenteral route.

5. Formulation according to claim 1 or 2, for reconstituting a solution for administration by oral route.

6. Formulation according to claim 4 for reconstituting an injectable solution.

7. Formulation according to claim 1 which can be administered directly by oral route.

8. Formulation according to claim 1, wherein the active principle is selected from among the phenylalkanoic acids, non-steroidal anti-inflammatories of the oxicam type, paracetamol, lysine or arginine acetylsalicylate, bile acids, corticosteroids, anthracyclines, phloroglucinol, platinum derivatives, alkaloid derivatives of *Vinca minor*, alkaloid derivatives of rye ergot, derivatives of purine or pyrimidine bases, prostaglandins, benzodiazepines, betalactam antibiotics, macrolide antibiotics, antibiotics of the tetracycline family, antibiotics of the chloramphenicol type, antibiotics of the spiramycin type, nitroso-ureas, nibrogenated mustards, H₂-antagonists, omeprazole, vitamins, antitumoral agents, cardiovascular drugs, haematological drugs, anticoagulant and antithrombotic agents, heparinoids, diarginine oxoglutarate, plant extracts, nucleotides, valproic acid analogues, metopimazine, moxisylyte, bisphosphonates acting as anti-osteoporotic agents, pralidoxime, deferoxamine, barbiturates, clomethiazole, 5-HT₂ antagonists, angiotensin II antagonists, fantofarone, tirapazamine, (2S)-1-[(2R,3S)-5-chloro-3-(2-chlorophenyl)-1-(3,4-dimethoxybenzenesulphonyl)-3-hydroxy-2,3-dihydro-1*H*-indole-2-carbonyl]-pyrrolidine-2-carboxamide, N,N-dibutyl-3-{4-[(2-butyl-5-methylsulphonamido)-benzofuran-3-yl-carbonyl]phenoxy}propylamine, 6-(2-diethylamino-2-methyl)propylamino-3-phenyl-4-propylpyridazine, ethyl{(7S)-7-[(2R)-2-(3-chlorophenyl)-2-hydroxyethylamino]-5,6,7,8-tetrahydro-naphthalen-2-yloxy}-acetate, 1-(2,4-dichlorophenyl)-3-(N-piperidin-1-yl-carboxamido)-4-methyl-5-(4-chlorophenyl)-1*H*-pyrazole, 4-{[N-(3,4-dimethoxy-phenethyl)]-N-methylaminopropoxyl}-2-benzenesulphonyl-3-isopropyl-1-methylindole, 2-{[1-(7-chloroquinolin-4-yl)-5-(2,6-dimethoxyphenyl)-1*H*-pyrazole-3-carbonyl]amino}adamantane-2-carboxylic acid, N-cyclohexyl-N-ethyl-3-(3-chloro-4-cyclohexylphenyl)prop-2-enylamine, (-) -N-methyl-N-[4-(4-acetylamino-4-phenylpiperidino)-2-(3,4-dichlorophenyl)butyl]benzamide, (S)-1-{2-[3-(3,4-dichlorophenyl)-1-(3-isopropoxyphenylacetyl)-piperidin-3-yl]ethyl}-4-phenyl-1-azoniabicyclo[2.2.2]octane chloride and the pharmaceutically acceptable quaternary salts thereof, 4-amino-1-(6-chloropyrid-2-yl)piperidine, (S) -N- (1-{3-[1-benzoyl-3-(3,4-dichlorophenyl)piperidin-3-yl]propyl}-4-phenylpiperidin-4-yl)-N-methylacetamide, 2-{[4-(2-chlorophenyl)thiazol-2-yl]aminocarbonyl}indole-1-acetic acid, clopidogrel, 1-(2-naphthalen-2-ylethyl)-4-(3-trifluoromethylphenyl)-1,2,3,6-tetrahydropyridine hydrochloride, N,N-dimethyl-N'-(pyridin-3-yl)methyl-N'-[4-(2,4,6-triisopropylphenyl)thiazol-2-yl]ethane-1,2-diamine, and the pharmaceutically acceptable salts thereof.

9. Formulation according to claim 1 wherein the active substance is selected from among 2-{[4-(2-chlorophenyl)thiazol-2-yl]aminocarbonyl}indole-1-acetic acid or the potassium salt thereof, irbesartan, clopidogrel, ursodesoxycholic acid and the sodium salt thereof, 1-(2-naphthalen-2-ylethyl)-4-(3-trifluoromethylphenyl)-1,2,3,6-tetrahydropyridine hydrochloride, N,N-dimethyl-N'-(pyridin-3-yl)methyl-N'-[4-(2,4,6-triisopropylphenyl)thiazol-2-yl]ethane-1,2-diamine fumarate, 2-[(5-(2,6-dimethoxyphenyl)-1-{4-[(3-dimethylaminopropyl)methylcarbamoyl]-2-isopropylphenyl}-1*H*-pyrazole-3-carbonyl)amino]adamantane-2-carboxylic acid, 3-(1-{2-[4-benzoyl-2-(3,4-difluorophenyl)morpholino-2-yl]ethyl}-4-phenylpiperidin-4-yl) -1, 1-dimethylurea, 3-[N-{4-[4-(aminoiminomethyl)phenyl]-1,3-thiazol-2-yl}-N-(1-carboxymethylpiperidin-4-yl)amino]propionic acid trihydrochloride, ethyl 3-[N-{4-[4-(amino(N-ethoxycarbonylimino)methyl)phenyl]-1,3-thiazol-2-yl}-N-(1-(ethoxycarbonylmethyl)piperidin-4-yl)amino]propionate, 5-ethoxy-1-[4-(N-tert.butylcarbamoyl)-2-methoxy-benzenesulphonyl]-3-spiro-[4-(2-morpholinoethyloxy)-cyclohexane]indolin-2-one and the pharmaceutically acceptable salts thereof.

10. Formulation according to claim 1 obtained after lyophilisation of a solution in which mannitol is present in a concentration of 9 mg per ml, alanine is present in a concentration of 18 mg per ml and the active substance is 2-{[4-(2-chlorophenyl)thiazol-2-yl]aminocarbonyl}indole-1-acetic acid in a concentration of 1.18 mg per ml or one of the pharmaceutically acceptable salts thereof in an equivalent concentration.

11. Formulation according to claim 1 obtained after lyophilisation of a solution in which mannitol is present in a concentration of 10 mg per ml, alanine is present in a concentration of 23 mg per ml and the active substance is irbesartan in a concentration of 1 mg per ml or one of the pharmaceutically acceptable salts thereof in an equivalent concentration.

12. Formulation according to claim 1 obtained after lyophilisation of a solution in which mannitol is present in a concentration of 9 mg per ml, alanine is present in a concentration of 18 mg per ml and the active substance is 1-(2-naphthalen-2-yl-ethyl)-4-(3-trifluoromethylphenyl)-1,2,3,6-tetrahydropyridine hydrochloride in a concentration of between 0.01 mg and 0.2 mg per ml or one of the pharmaceutically acceptable salts thereof in an equivalent concentration.

13. Process for stabilising a non-protein active substance contained in a pharmaceutically acceptable lyophilised formulation, comprising steps consisting of:
- dissolving the non-protein active substance, mannitol and alanine in a ratio R (of mannitol to alanine) of between 0.1 and 1 in water for injections or in a solubilising agent, at the appropriate dissolution temperature, optionally in the presence of a buffer and preservatives;
- filtering the solutions obtained; and
- lyophilising the resulting solutions by carrying out a cycle of freezing followed by sublimation and drying.
